# EUROPEAN PATENT APPLICATION

(11) **EP 3 492 065 A1**
(43) Date of publication of application: **05.06.2019**
(21) Application number: 17204953.8
(22) Date of filing: 01.12.2017
(51) Int. Cl.: A61K 8/35, A61K 8/06, A61K 8/46, A61K 8/49, A61Q 19/08, A61K 8/39

(54) **COMPOSITION FOR TOPICAL SKIN CARE**

(71) Applicant: NCP NewCare Products GmbH, 22523 Hamburg (DE)
(72) Inventor: KRAINBRING, Volker Gustav, 25451 Quickborn (DE)
(74) Representative: Uexküll & Stolberg

(57) **Abstract**

The present invention relates to a novel topical composition for treatment of the skin. The composition is a microemulsion that comprises curcumin or a curcumin derivative. The composition is able to penetrate into skin cells where it induces the production of adenosine triphosphate (ATP) and promotes the formation of new skin cells. The formation of new skin cells smoothes the skin and alleviates lines and wrinkles. The topical composition can be applied to the skin of a person daily. The invention also relates to the use of the novel topical composition for delaying skin aging.

## Description

The present invention relates to a novel topical composition for treatment of the skin. The composition is a microemulsion that comprises curcumin or a curcumin derivative. The composition is able to penetrate into skin cells where it induces the production of adenosine triphosphate (ATP) and promotes the formation of new skin cells. The formation of new skin cells smoothes the skin and alleviates lines and wrinkles. The topical composition can be applied to the skin of a person daily. The invention also relates to the use of the novel topical composition for delaying skin aging.

### BACKGROUND

Skin aging is a complex process that is caused by a number of different intrinsic and extrinsic factors. For example, it is long known that the exposure to ultraviolet (UV) light promotes wrinkling, a loss of skin elasticity, erythema, hyper-pigmentation, and skin cancer.

Amongst others, UV-A radiation has been shown to cause oxidative damage in nuclear and mitochondrial DNA by producing free radicals. UV-A radiation furthermore induces inflammatory responses that lead to elevated levels of matrix metalloproteinases in human skin. The matrix metalloproteinases degrade collagen which results in a disorganization of collagen fibrils in the dermal connective tissue. Ultimately, this leads to loss of skin elasticity and to the formation of wrinkles. UV-B radiation is known to produce non-oxidative DNA damage by linking adjacent bases in the DNA so as to form cyclobutane pyrimidine dimers (CPD). These dimers can be formed between two adjacent pyrimidines and are highly mutagenic.

Apart from such extrinsic factors, it has been found that also intrinsic factors, like the skin energy metabolism, contribute to the loss of skin function and therefore contribute to the aging process. The skin comprises numerous types of proliferating cells, wherein the cells have a high energy demand. On the molecular level, adenosine triphosphate (ATP) represents the source of energy. ATP is produced in the mitochondria of the cells. With increasing age, the activity of the mitochondria decreases which means that less ATP is produced that can be consumed by the cells in the skin. However, ATP is required for cell proliferation, DNA damage repair, and collagen synthesis. The decrease in ATP production therefore directly affects the skin function and ultimately also the appearance of the skin.

For the above reason, it has been contemplated to delay skin aging by increasing the ATP level within the cells. A number of currently available skincare products contain ATP as an ingredient. However, the effectiveness of such skincare products is rather low, because it has proven to be highly problematic for the ATP contained in topical compositions to reach the skin cells and to be taken up by them.

Accordingly, there is a need in the prior art for means and methods that allow for an increase of the ATP levels in skin cells. Such means and methods would be highly desirable for ameliorating or delaying the symptoms of skin aging.

### DETAILED DESCRIPTION OF THE INVENTION

The present invention provides a novel cosmetically active composition in the form of a microemulsion which is able to effectively permeate into skin cells like keratinocytes, melonacytes and fibroblasts. The composition comprises an effective amount of curcumin or a curcumin derivative. It has been found that the delivery of curcumin or a curcumin derivative to the skin cells is effective in increasing the ATP level in skin cells of ATP. Since a sufficient amount of ATP in the skin cells is required for maintaining the integrity of the collagen network, the increase in ATP within the cell is an appropriate means for preventing the symptoms of skin aging, i.e. the formation of wrinkles.

It has been found that by using three different types of surfactants, microemulsions can be formed that show particularly good skin penetration properties. The microemulsions are capable of penetrating into different skin cells like keratinocytes and melanocytes where the curcumin or curcumin derivative enhances ATP production.

The cosmetic composition of the invention is a microemulsion comprising the following components:
(a) an alcohol ethoxysulfate;
(b) an ethoxylated glyceryl fatty acid ester;
(c) an ethoxylated sorbitol or sorbitol anhydride fatty acid ester; and
(d) curcumin or a curcumin derivative.

The cosmetic composition of the invention comprises at least the three surfactants in amounts that allow the formation of a microemulsion. As used herein, a microemulsion refers to a macroscopically homogeneous mixture of water, oil and one or more surfactants. In the compositions of the present invention, one of the three surfactants can represent the oil component. Preferably, the PEG fatty acid ester may form the oil component of the composition.

As a first component, the composition of the invention comprises an alcohol ethoxysulfate (AES). Alcohol ethoxysulfates are a class of surfactants that is widely used in cosmetic and cleaning products. As used herein, the alcohol ethoxysulfate for use in the composition of the invention has the general formula (I) R'-O-(CH₂-CH₂O)ₙ-SO₃M in which R' is an alkyl group or an alkylaryl group, n represents the average number of oxyethylene groups per molecule, and M is a cation.

In a preferred embodiment, the alcohol ethoxysulfate has the above formula (I) in which R' is a straight or branched alkyl group with 8-18 carbon atoms, or an alkylaryl group having an alkyl moiety having from 8 to 12 carbon atoms, n represents the average number of oxyethylene groups per molecule which is from 1 to 12, and M is a cation selected from an alkali metal ion, such as Na+ or Ka+, an ammonium ion, and mixtures thereof.

In another preferred embodiment, the alcohol ethoxysulfate has the above formula (I) in which R' is a straight or branched alkyl group with 8-18 carbon atoms, n represents the average number of oxyethylene groups per molecule which is from 1 to 8, and M is a cation selected from an alkali metal ion, such as Na+ or Ka+, an ammonium ion, and mixtures thereof.

In yet another preferred embodiment, the alcohol ethoxysulfate has the above formula (I) in which R' is a straight or branched alkyl group with 10-14 carbon atoms, n represents the average number of oxyethylene groups per molecule which is from 1 to 4, and M is a cation selected from an alkali metal ion, such as Na+ or Ka+, an ammonium ion, and mixtures thereof.

In yet another preferred embodiment, the alcohol ethoxysulfate has the above formula (I) in which R' is a straight alkyl group with 10-14 carbon atoms, n represents the average number of oxyethylene groups per molecule which is from 2 to 3, and M is a cation selected from an alkali metal ion.

In a particularly preferred embodiment, the alcohol ethoxysulfate has the above formula (I) in which R' is a straight alkyl group with 10-12 carbon atoms, n represents the average number of oxyethylene groups per molecule which is from 2 to 3, and M is a cation selected from Na+ or Ka+.

The alcohol ethoxysulfate is preferably a dodecyl alcohol ethoxysulfate. In a particularly preferred embodiment, the alcohol ethoxysulfate in the composition of the invention is sodium lauryl ether sulfate.

The alcohol ethoxysulfate is preferably present in the composition in an amount of about 0.5 to about 25.0% (w/w). This means that the alcohol ethoxysulfate can be present in an amount ranging from about 1.0 to about 20.0% (w/w), from about 1.0 to about 15.0% (w/w), from about 1.0 to about 10.0% (w/w), from about 1.0 to about 5.0% (w/w), from about 2.0 to about 25.0% (w/w), from about 2.0 to about 20.0% (w/w), from about 2.0 to about 15.0% (w/w), from about 2.0 to about 10.0% (w/w), from about 2.0 to about 5.0% (w/w), from about 3.0 to about 25.0% (w/w), from about 3.0 to about 20.0% (w/w), from about 3.0 to about 15.0% (w/w), from about 3.0 to about 10.0% (w/w), from about 3.0 to about 5.0% (w/w), from about 4.0 to about 25.0% (w/w), from about 4.0 to about 20.0% (w/w), from about 4.0 to about 15.0% (w/w), from about 4.0 to about 10.0% (w/w), or from about 4.0 to about 5.0% (w/w).

As used herein, the term "about", when used in combination with a numeric value shall mean ±10% of said recited value.

In a particularly preferred embodiment, the alcohol ethoxysulfate is present in the composition in an amount of about 4.5 to about 5.5%, such as about 5% (w/w). In a particularly preferred embodiment, the composition of the invention comprises sodium lauryl ether sulfate in an amount of about 4.5 to about 5.5% (w/w), more preferably about 5% (w/w).

As a second component, the composition of the present invention comprises an ethoxylated glyceryl fatty acid ester. Ethoxylated glyceryl fatty acid esters are esters of glycerol with one or more fatty acids to which polyethylene glycol has been added. While the hydrophilic polyethylene glycol part of the molecule is hydrophilic, the fatty acid residue is lipophilic. Owing to their amphiphilic properties, ethoxylated glyceryl fatty acid esters are commonly used as surfactants in cosmetic products.

The number of ethylene glycol groups in the molecule is not particularly relevant. The ethoxylated glyceryl esters for use in the present invention may comprise about 1-600 repeating ethylene glycol groups, such as about 1-500, about 1-400, about 1-300, about 1-200, about 1-100, about 1-90, about 1-80, about 1-70, about 1-60, about 1-50, about 1-40, about 1-30, about 1-20, about 1-10, or about 1-5. An ethoxylated glyceryl ester with 1-10 ethylene glycol groups is particularly preferred. In a preferred embodiment, the ethoxylated glyceryl ester comprises 7 ethylene glycol groups.

The ethoxylated glyceryl ester can be a mono-, di-, or triester, or a mixture of those. In a preferred embodiment, the ester is a monoester. The ethoxylated glyceryl ester may be an ester of glycerol with saturated or unsaturated fatty acids. Suitable saturated fatty acids include caprylic acid (C8), capric acid (C10), lauric acid (C12), myristic acid (C14), palmitic acid (C16), stearic acid (C18), arachidic acid (C20), behenic acid (C22), lignoceric acid (C24) and cerotic acid (C26). Suitable unsaturated fatty acids include myristoleic acid (C14), palmitoleic acid (C16), sapienic acid (C16), oleic acid (C18), elaidic acid (C18), vaccenic acid (C18), linoleic acid (C18), arachidonic acid (C20), eisopentaenoic acid (C20), erucic acid (C22), and docohexaenoic acid (C22).

In one preferred embodiment, the ethoxylated glyceryl ester is an ethoxylated glyceryl cocoate, i.e. an ester of glycerol with coconut-oil derived fatty acids. In another preferred embodiment, the ethoxylated glyceryl ester is an ethoxylated glyceryl cocoate monoester. Preferably, the ethoxylated glyceryl cocoate contains from 1-80, from 1-40, from 1-20, from 1-10 or from 1-7 repeating ethylene glycol groups. Examples for suitable cocoate esters include, but are not limited to, PEG-7 glyceryl cocoate, PEG-30 glyceryl cocoate, PEG-40 glyceryl cocoate, PEG-78 glyceryl cocoate and PEG-80 glyceryl cocoate. In a particularly preferred embodiment, the ethoxylated glyceryl cocoate is PEG-7 glyceryl cocoate, which is commercially available, for instance, as Cetiol HE (BASF, Ludwigshafen, Germany).

The ethoxylated glyceryl fatty acid ester is preferably present in the composition in an amount of 1.0 to 50.0% (w/w). This means that the ethoxylated glyceryl fatty acid ester can be present in a range from about 1.0 to about 45.0% (w/w), from about 1.0 to about 40.0% (w/w), from about 1.0 to about 35.0% (w/w), from about 1.0 to about 30.0% (w/w), from about 1.0 to about 25.0% (w/w), from about 1.0 to about 20.0% (w/w), from about 1.0 to about 15.0% (w/w), from about 1.0 to about 10.0% (w/w), from about 5.0 to about 45.0% (w/w), from about 5.0 to about 40.0% (w/w), from about 5.0 to about 35.0% (w/w), from about 5.0 to about 30.0% (w/w), from about 5.0 to about 25.0% (w/w), from about 5.0 to about 20.0% (w/w), from about 5.0 to about 15.0% (w/w), from about 5.0 to about 10.0% (w/w), from about 7.5 to about 45.0% (w/w), from about 7.5 to about 40.0% (w/w), from about 7.5 to about 35.0% (w/w), from about 7.5 to about 30.0% (w/w), from about 7.5 to about 25.0% (w/w), from about 7.5 to about 20.0% (w/w), from about 7.5 to about 15.0% (w/w), or from about 7.5 to about 10.0% (w/w). In a preferred embodiment, the ethoxylated glyceryl fatty acid ester is present in the composition in an amount of about 7.5 to about 15.0%, such as about 10% (w/w).

In a preferred embodiment, the ethoxylated glyceryl fatty acid ester is present in the composition in an amount of 9.5 to 10.5%, such as 10.0% (w/w). In an even more preferred embodiment, the composition of the invention comprises an ethoxylated glyceryl cocoate in an amount of 9.5 to 10.5% (w/w), more preferably 10.0% (w/w). In a most preferred embodiment, the composition of the invention comprises PEG-7 glyceryl cocoate in an amount of 9.5 to 10.5% (w/w), more preferably 10.0% (w/w).

As a third component, the composition of the invention comprises an ethoxylated sorbitol or sorbitol anhydride fatty acid ester. These esters are based on the polyhydric sugar alcohol sorbitol or its anhydrides, e.g. sorbitan. Preferably, the ethoxylated sorbitol or sorbitol anhydride fatty acid ester is a partial ester which means that part of the hydroxyl groups in the sorbitol or sorbitol anhydride are not esterified. The sorbitol or sorbitol anhydride ester may be an ester with any type of saturated or unsaturated fatty acids.

Suitable saturated fatty acids include caprylic acid (C8), capric acid (C10), lauric acid (C12), myristic acid (C14), palmitic acid (C16), stearic acid (C18), arachidic acid (C20), behenic acid (C22), lignoceric acid (C24) and cerotic acid (C26). Suitable unsaturated fatty acids include myristoleic acid (C14), palmitoleic acid (C16), sapienic acid (C16), oleic acid (C18), elaidic acid (C18), vaccenic acid (C18), linoleic acid (C18), arachidonic acid (C20), eisopentaenoic acid (C20), erucic acid (C22), and doco-hexaenoic acid (C22).

In a preferred embodiment, the ethoxylated sorbitol or sorbitol anhydride fatty acid ester is an oleic acid, stearic acid, or lauric acid ester. A partial ester of sorbitol with oleic acid, stearic acid, or lauric acid is most preferred. The composition may comprise a mixture of different esters or partial esters of sorbitol or sorbitol anhydride with different fatty acids, such as a mixture of different partial esters of sorbitol or a sorbitol anhydride with oleic acid, stearic acid, or lauric acid.

Preferably, the ethoxylated sorbitol or sorbitol anhydride ester contains about 1-85, about 1-40, about 1-20, about 1-10 or about 1-5 ethylene glycol groups.

It is particularly preferred that the ethoxylated sorbitol or sorbitol anhydride ester is selected from the group consisting of polysorbate 20, polysorbate 21, polysorbate 40, polysorbate 60, polysorbate 61, polysorbate 65, polysorbate 80, polysorbate 81, polysorbate 85, and polysorbate 120. Polysorbate 80 is particularly preferred.

The ethoxylated sorbitol or sorbitol anhydride ester is preferably present in the composition in an amount of 0.5 to 25.0% (w/w). This means that the ethoxylated sorbitol or sorbitol anhydride ester can be present in a range from about 1.0 to about 20.0% (w/w), from about 1.0 to about 15.0% (w/w), from about 1.0 to about 10.0% (w/w), from about 1.0 to about 8.0% (w/w), from about 2.0 to about 25.0% (w/w), from about 2.0 to about 20.0% (w/w), from about 2.0 to about 15.0% (w/w), from about 2.0 to about 10.0% (w/w), from about 2.0 to about 8.0% (w/w), from about 3.0 to about 25.0% (w/w), from about 3.0 to about 20.0% (w/w), from about 3.0 to about 15.0% (w/w), from about 3.0 to about 10.0% (w/w), from about 3.0 to about 8.0% (w/w), from about 4.0 to about 25.0% (w/w), from about 4.0 to about 20.0% (w/w), from about 4.0 to about 15.0% (w/w), from about 4.0 to about 10.0% (w/w), or from about 4.0 to about 8.0% (w/w). In a most preferred embodiment, the ethoxylated sorbitol or sorbitol anhydride ester is preferably present in the composition in an amount of about 6.0 to about 9.0%, such as about 8.0% (w/w).

In a preferred embodiment, the composition of the invention comprises an ethoxylated sorbitol ester in an amount of about 6.0 to about 9.0%, such as about 8.0% (w/w). In an even more preferred embodiment, the composition comprises a polysorbate in an amount of about 9.5 to about 10.5% (w/w), more preferably about 10.0% (w/w). In a most preferred embodiment, the composition of the invention comprises Polysorbate 80 in an amount of about 9.5 to about 10.5% (w/w), more preferably about 10.0% (w/w).

It is preferred that the weight ratio of ethoxylated glyceryl fatty acid ester/alcohol ethoxysulfate/ethoxylated sorbitol or sorbitol anhydride fatty acid ester is approximately 3:2:1, more preferably 2.5:1.8:1 and even more preferably 2:1.6:1.

The Composition further comprises curcumin or a curcumin derivative. Curcumin is a linear diarylheptanoid compound that is produced by turmeric (Curcuma longae), a member of the ginger family. Curcumin has the following structure:

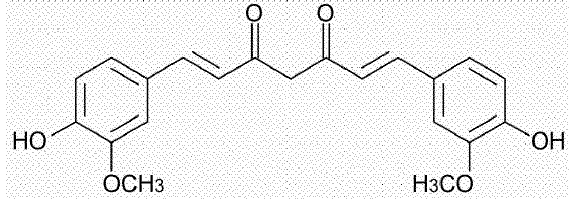

Several derivatives of curcumin have been described in the art and can likewise be used in the compositions of the invention as well, such as desmethoxycurcumin or bisdemethoxycurcumin.

The curcumin or curcumin derivative is preferably present in the composition in an amount of about 0.0001 to about 0.05% (w/w), preferably, about 0.001 to about 0.03% (w/w), about 0.002 to about 0.01% (w/w), and more preferably about 0.0025 (w/w). According to the invention, a curcumin-containing extract can be included into the composition, such as an extract of Curcuma longae. The extract may be a tincture, i.e. an extract that was prepared by maceration or percolation. Preferably the extract will be an alcoholic extract, such as an ethanol extract. Where an extract or tincture is used, the amount of the extract or tincture will be chosen to correspond to an amount of 0.0001 to 0.05% (w/w) pure curcumin. Of course, one can also use isolated curcumin.

Apart from the surfactants and curcumin, the compositions of the invention may comprise further components that are common ingredients in cosmetic products. For example, the compositions of the invention may comprise urea. When used in small amounts, urea has beneficial water-binding and mild exfoliating properties for the skin and enhances the absorption of other cosmetic ingredients.

The compositions may also comprise an acid for adapting the pH. Normally, the compositions of the invention will have a slightly acidic pH to make it more compatible with the acidic environment of the skin. The compositions may have a pH in the range from about 2.5 to about 6.5, preferably from about 4.0 to about 6.0, and more preferably from about 5.0 to about 6.0 or from about 5.5 to about 6.0. The acidic pH can be achieved by adding an acid to the microemulsion, e.g. a carboxylic acid, such as an alpha hydroxy acid. The nature of the acid that can be used in the composition of the invention is not limited. Suitable acids include formic acid, acetic acid, propionic acid, butyric acid, valeric acid, caproic acid, enanthic acid, caprylic acid, and the like. For application to the human skin, lactic acid is particularly useful, as it is also secreted by the skin flora to form the protective acidic milieu on the human skin surface.

The composition for skin treatment may comprise further additives for improving hydration of the skin, in particular a humectant. Suitable humectants for use in the composition of the present invention include amino acids, e.g. proline and arginine, glucuronic acid salts, glutamic acid salts, glycerine, polyethylene glycol ethers of glycerine, sugar and sugar alcohols, e.g. glucose, mannose and polyglycerol sorbitol, trehalose, hydrogenated starch hydrolysates, hydrolyzed mucopolysaccharides, inositol, and the like. The composition of the invention may comprise from about 1% to about 15% (w/w) of a humectant. In some embodiments, the composition may contain two or more different humectants.

In a particularly preferred embodiment, the composition of the invention further comprises an Aloe vera extract. Preferably, the extract may be an Aloe vera from the leaves of the plant Aloe vera which is widely used in cosmetics for its moisturising and revitalising properties (see Martindale, The Complete Drug Reference, 34th Edition, Sean C Sweetman Ed., Pharmaceutical Press (2005)). Aloe vera extract contains a high amount of sugars which, after delivery to the skin cells, are consumed by the cells in the citrate cycle to produce ATP. In this way, the Aloe vera extract is able to enhance the effect exerted by curcumin.

In one embodiment, the Aloe vera used in the cosmetic composition of the invention herein is an Aloe vera gel. Aloe vera gel is commercially available as Aloe vera leaf juice both in unconcentrated and concentrated form. Where a concentrate is used in the composition of the invention, the concentrate is preferably diluted with water to achieve the equivalent of 100% Aloe vera gel. This means, the concentrate is diluted to mimic the plant juice in its unconcentrated form. The quantity of Aloe vera concentrate used in the compositions of the invention will vary according to the concentrate used. For example, where a 2-fold concentrate is used in the compositions of the invention, the Aloe vera concentrate may be present in an amount from about 10% to about 50% (w/w) . Where a 5-fold concentrate is used, the concentrate may be present in an amount from about 4% to about 20% (w/w). Where a 10-fold concentrate is used, the concentrate may be present in an amount from about 2% to about 10% (w/w). Where a 20-fold concentrate is used, the concentrate may be present in an amount from about 1% to about 5% (w/w). Where a 40-fold concentrate is used, the concentrate may be present in an amount from about 0.5% to about 2.5% (w/w). Where a 200-fold concentrate is used, the Aloe vera concentrate may present in an amount from about 0.1% to about 0.5% (w/w). It is preferred that the Aloe vera concentrate is a 10-fold concentrate which is present in an amount of about 10.0% (w/w).

Other components that are commonly used in the field of cosmetics may be added to the composition as well, such as abrasives, antifoaming agents, antimicrobial agents, binders, buffering agents, bulking agents, denaturants, film formers, fragrances, pigments, emollients, propellants, skin penetration enhancing agents, solvents, thickening agents, antioxidants or radical scavengers, chelating agents, anti-inflammatory agents, vitamins and vitamin derivatives, and the like. Such components are described, for example, in The Handbook of Cosmetic Science and Technology, 1st Ed., Knowlton & Pearce (Elsevier 1993).

In a preferred embodiment, the cosmetic composition of the invention is a microemulsion comprising the following components:
(a) an alcohol ethoxysulfate in an amount of about 0.5 to about 25.0% (w/w),
(b) an ethoxylated glyceryl fatty acid ester in an amount of about 1.0 to about 50.0% (w/w),
(c) an ethoxylated sorbitol or sorbitol anhydride fatty acid ester in an amount of about 0.5 to about 25.0% (w/w),
(d) curcumin or a curcumin derivative 0.0001 to about 0.05% (w/w), and
(e) optionally, an Aloe vera extract in an amount of about 0.1 to about 15.0% (w/w).

In another preferred embodiment, the cosmetic composition of the invention is a microemulsion comprising the following components:
(a) an alcohol ethoxysulfate in an amount of about 0.5 to about 20.0% (w/w),
(b) an ethoxylated glyceryl fatty acid ester in an amount of about 1.0 to about 40.0% (w/w),
(c) an ethoxylated sorbitol or sorbitol anhydride fatty acid ester in an amount of about 0.5 to about 30.0% (w/w),
(d) curcumin or a curcumin derivative in an amount of about 0.0001 to about 0.05% (w/w), and
(e) optionally, an Aloe vera extract in an amount of about 0.5 to about 12.0% (w/w).

In another preferred embodiment, the cosmetic composition of the invention is a microemulsion comprising the following components:
(a) an alcohol ethoxysulfate in an amount of about 0.5 to about 15.0% (w/w),
(b) an ethoxylated glyceryl fatty acid ester in an amount of about 1.0 to about 30.0% (w/w),
(c) an ethoxylated sorbitol or sorbitol anhydride fatty acid ester in an amount of about 0.5 to about 24.0% (w/w),
(d) curcumin or a curcumin derivative in an amount of about 0.0001 to about 0.05% (w/w), and
(e) optionally, an Aloe vera extract in an amount of about 0.5 to about 10.0% (w/w).

In yet another preferred embodiment, the cosmetic composition of the invention is a microemulsion comprising the following components:
(a) an alcohol ethoxysulfate,
(b) an ethoxylated glyceryl cocoate,
(c) a polysorbate,
(d) curcumin or a curcumin derivative, and
(e) optionally, an Aloe vera extract.

In yet another preferred embodiment, the cosmetic composition of the invention is a microemulsion comprising the following components:
(a) sodium lauryl ether sulfate,
(b) PEG-7 glyceryl cocoate,
(c) polysorbate 80,
(d) curcumin or a curcumin derivative, and
(e) optionally, an Aloe vera extract.

In yet another preferred embodiment, the cosmetic composition of the invention is a microemulsion comprising the following components:
(a) an alcohol ethoxysulfate in an amount of about 0.5 to about 25.0% (w/w),
(b) an ethoxylated glyceryl cocoate in an amount of about 1.0 to about 50.0% (w/w),
(c) a polysorbate in an amount of about 0.5 to about 25.0% (w/w),
(d) curcumin or a curcumin derivative, and
(e) optionally, an Aloe vera extract in an amount of about 0.1 to about 15.0% (w/w).

In yet another preferred embodiment, the cosmetic composition of the invention is a microemulsion comprising the following components:
(a) sodium lauryl ether sulfate in an amount of about 0.5 to about 25.0% (w/w),
(b) PEG-7 glyceryl cocoate in an amount of about 1.0 to about 50.0% (w/w),
(c) polysorbate 80 in an amount of about 0.5 to about 25.0% (w/w),
(d) curcumin or a curcumin derivative in an amount of about 0.0001 to about 0.05% (w/w), and
(e) optionally, an Aloe vera extract in an amount of about 0.1 to about 15.0% (w/w).

In yet another preferred embodiment, the cosmetic composition of the invention is a microemulsion comprising the following components:
(a) sodium lauryl ether sulfate in an amount of about 0.5 to about 20.0% (w/w),
(b) an ethoxylated glyceryl cocoate in an amount of about 1.0 to about 40.0% (w/w),
(c) a polysorbate in an amount of about 0.5 to about 30.0% (w/w),
(d) curcumin or a curcumin derivative in an amount of about 0.0001 to about 0.05% (w/w), and
(e) optionally, an Aloe vera extract in an amount of about 0.5 to about 12.0% (w/w).

In yet another preferred embodiment, the cosmetic composition of the invention is a microemulsion comprising the following components:
(a) sodium lauryl ether sulfate in an amount of about 0.5 to about 20.0% (w/w),
(b) PEG-7 glyceryl cocoate in an amount of about 1.0 to about 40.0% (w/w),
(c) polysorbate 80 in an amount of about 0.5 to about 30.0% (w/w),
(d) curcumin or a curcumin derivative in an amount of about 0.0001 to about 0.05% (w/w), and
(e) optionally, an Aloe vera extract in an amount of about 0.5 to about 12.0% (w/w).

In yet another preferred embodiment, the cosmetic composition of the invention is a microemulsion comprising the following components:
(a) sodium lauryl ether sulfate in an amount of about 0.5 to about 15.0% (w/w),
(b) an ethoxylated glyceryl cocoate in an amount of about 1.0 to about 30.0% (w/w),
(c) a polysorbate in an amount of about 0.5 to about 24.0% (w/w),
(d) curcumin or a curcumin derivative in an amount of about 0.0001 to about 0.05% (w/w), and
(e) optionally, an Aloe vera extract in an amount of about 0.5 to about 10.0% (w/w).

In yet another preferred embodiment, the cosmetic composition of the invention is a microemulsion comprising the following components:
(a) sodium lauryl ether sulfate in an amount of 15.0% (w/w),
(b) PEG-7 glyceryl cocoate in an amount of about 30.0% (w/w),
(c) polysorbate 80 in an amount of about 24.0% (w/w),
(d) curcumin or a curcumin derivative in an amount of 0.0025% (w/w), and
(e) optionally, an Aloe vera extract in an amount of about 10.0% (w/w).

A cosmetic composition as described above is suitable for being used in skin care treatment. The compositions that are to be administered to the skin will typically be formulated to allow their transdermal delivery. For this, the microemulsions for skin treatment can be directly administered topically to the skin area to be treated. For example, the microemulsions referred to above can be packaged into dropping bottles that allow the dripping of the microemulsion directly onto the skin area in need of treatment. Alternatively, the compositions can be packaged into pump dispensers which allow spraying the microemulsion onto the skin areas to be treated. The microemulsions may also be formulated into ointments, creams, suspensions, lotions, pastes, gels, hydrogels, foams or oils. The microemulsions can also be incorporated into patches.

Accordingly, the cosmetic composition is suitable for delaying the symptoms of skin aging, such as wrinkles and lines. The cosmetic composition is also suitable for improving skin elasticity and overall skin appearance. The cosmetic composition is also suitable for improving skin tone and skin evenness. Thus, in one aspect, the invention relates to the use of a cosmetic composition as described above for improving skin elasticity or skin appearance. In another aspect, the invention relates to the use of a cosmetic composition as described above for delaying the symptoms of skin aging.

The compositions may be applied to the area of skin in need of treatment at least once a day, twice a day, or even more frequently. When applied twice daily, the first and second applications are separated by at least 6 hours, preferably 8 hours. Typically, the cosmetic composition is applied once in the morning and once in the evening. The period in which the composition is applied to the skin will be such that an improvement in the appearance of the skin, preferably, with respect to wrinkles and lines, skin elasticity, skin tone or skin evenness is determined. The period of treatment may be at least 1 week, at least 2 weeks, at least 3 weeks, at least 4 weeks, at least 6 weeks, at least 12 weeks, at least 24 weeks, or more. In some embodiments, the treatment will be extended for several months, such as for 4 months, 6 months, 8 months, 12 months, 18 months, or 24 months.

Finally, the invention also provides a cosmetic method for improving skin elasticity or skin appearance, or delaying the symptoms of skin aging, said method comprising topically applying the cosmetic composition as described herein above.

### EXAMPLES

The following examples describe certain preferred embodiments of the present invention. It is however to be noted that the invention is not limited to such embodiments.

### Example 1: Composition for delaying skin aging

A composition for delaying the symptoms of skin aging was prepared as followed:

| | |
|---|---|
| 1. Cetiol HE | 30.0 g |
| 2. Polysorbat 80 | 24.0 g |
| 3. Texapon NSO | 15.0 g |
| 4. Aloe Vera (1:10 extract), 70% ethanol) | 10.0 g |
| 5. Ethanol (70%) | 6.0 g |
| 6. Tincture Curcuma longae (1:10 in 70% ethanol) | 1.0 g |
| 7. Euxyl K712 | 0.1 g |
| 8. Aqua dem. | ad 100 g |

Components 1 to 3 were mixed by slowly stirring at room temperature to provide pre-mix A. At the same time, components 4 to 7 were mixed by stirring at room temperature to provide pre-mix B. Pre-mix A and pre-mix B were mixed under slow stirring to result in a transparent microemulsion having a pH of 5.5. The microemulsion was filled into a cosmetic pump dispenser having a volume of 100 ml.

### Example 2: Skin care treatment

The microemulsion of Example 1 was tested with 7 test persons over a period of 4 weeks. Photographs were taken before and after the treatment period. The microemulsion of Example 1 was applied twice daily by applying it to the face of the test person. After the 4-weeks treatment, the skin was visually evaluated.

Results: After 4 weeks of treatment, all test persons showed a significantly smoother and firmer skin. In particular, wrinkles around the eyes had significantly decreased. The skin of all test persons appeared more toned.

## Claims

1. Cosmetic composition in the form of a microemulsion, comprising
(a) an alcohol ethoxysulfate;
(b) an ethoxylated glyceryl fatty acid ester;
(c) an ethoxylated sorbitol or sorbitol anhydride fatty acid ester;
(d) curcumin or a curcumin derivative.

2. Cosmetic composition according to claim 1, wherein said alcohol ethoxysulfate is dodecyl alcohol ethoxysulfate, preferably sodium lauryl ether sulfate.

3. Cosmetic composition according to any of claims 1-2, wherein said alcohol ethoxysulfate is present in the composition in an amount of about 0.5 to about 25.0% (w/w), preferably about 5% to about 10.0% (w/w).

4. Cosmetic composition according to any of claims 1-3, wherein said ethoxylated glyceryl fatty acid ester is an ethoxylated glyceryl cocoate, preferably PEG-7 glyceryl cocoate.

5. Cosmetic composition according to any of claims 1-4, wherein said ethoxylated glyceryl fatty acid ester is present in the composition in an amount of 1.0 to 50.0% (w/w), preferably about 5% to about 20.0% (w/w).

6. Cosmetic composition according to any of claims 1-5, wherein said ethoxylated sorbitol or sorbitol anhydride fatty acid ester is a partial ester with oleic acid, preferably polysorbat 80.

7. Cosmetic composition according to any of claims 1-6, wherein said ethoxylated sorbitol or sorbitol anhydride ester is present in the composition in an amount of about 0.5 to about 25.0% (w/w), preferably about 5% to about 10.0% (w/w).

8. Cosmetic composition according to any of claims 1-7, wherein said curcumin or a curcumin derivative is present in the composition in an amount of about 0.0001 to about 0.05% (w/w).

9. Cosmetic composition according to any of claims 1-8, wherein said composition is formulated as a creme, lotion or ointment for topical application.

10. Cosmetic composition according to any of claims 1-9, wherein said composition further comprises an Aloe vera extract.

11. Cosmetic composition according to any of claims 1-10, wherein said Aloe vera extract is present in the composition in an amount of about 0.1 to about 15.0% (w/w).

12. Use of a cosmetic composition according to any of claims 1-11 for improving skin elasticity or skin appearance.

13. Use of a cosmetic composition according to any of claims 1-11 for delaying the symptoms of skin aging.

14. Cosmetic method for improving skin elasticity or skin appearance, or delaying the symptoms of skin aging, said method comprising topically applying the cosmetic composition of any of claims 1-11.

15. Cosmetic method of claim 14, wherein said cosmetic composition is applied to the face.
